# EUROPEAN PATENT SPECIFICATION

(11) **EP 0 735 885 B1**
(45) Date of publication and mention of the grant of the patent: **28.07.2004**
(21) Application number: 95905880.1
(22) Date of filing: 19.12.1994
(51) Int. Cl.: A61P 17/00

(54) **OINTMENTS COMPRISING MOMETASONE FUROATE AND SALICYLIC ACID FOR THE TOPICAL TREATMENT OF PSORIASIS**
SALBE ENTHALTEND MOMETASON-FUROAT UND SALICYLSÄURE ZUR TOPISCHEN BEHANDLUNG VON PSORIASIS
ONGUENT TOPIQUE CONTENANT DU FUROAT DE MOMETASONE AT DE L'ACIDE SALICYLIQUE POUR LE TRAITEMENT DU PSORIASIS

(30) Priority: 21.12.1993 US 171051
(43) Date of publication of application: 09.10.1996
(73) Proprietor: SCHERING CORPORATION, Kenilworth, New Jersey 07033-0530 (US)
(72) Inventor: SEQUEIRA, Joel A., Scotch Plains, NJ 07076 (US); CHAUDRY, Imtiaz A., North Caldwell, NJ 07006 (US); PEETS, Edwin A., New York, NY 10025 (US); TANNER, Daniel J., Brooklyn, NY 11219 (US); TAYLOR, Eugene L., Bernardsville, NJ 07924 (US)
(74) Representative: Ritter, Stephen David
(86) International application number: PCT/US1994/014165
(87) International publication number: WO 1995/017196

(56) References cited:
- EP-A- 0 262 681
- EP-A- 0 292 893
- WO-A-91/07169
- DE-A- 2 744 826
- US-A- 4 472 393
- US-A- 4 579 844
- US-A- 5 004 736

## Description

### BACKGROUND OF THE INVENTION

This invention relates to topical pharmaceutical compositions preferably in the form of an ointment for treating psoriasis and other hyperkeratotic and/or scaly dermatoses which comprise mometasone furcate and salicylic acid together with specific pharmaceutically acceptable carriers. Methods of treating psoriasis and other hyperkeratotic and/or scaly dermatoses using such topical pharmaceutical compositions are also disclosed.

Mometasone furoate is a synthetic corticosteroid with anti-inflammatory activity available from Schering Corporation, Kenilworth, New Jersey, under the trademark ELOCON Cream, Lotion and Ointment and indicated for relief of the inflammatory and pruritic manifestations of corticosteroid-responsive dermatoses.

EP-A-0262681 and US-A-4472393 disclose topical compositions with mometasone furoate and their use as anti-inflammatory agents.

DE-A-2744826 describes topical compositions for the treatment of dermatoses containing corticosteroids and salicylic acid.

US-A-5004736 discloses topical compositions for the treatment of psoriasis containing corticosteroids and salicylic acid.

Salicylic acid is a commercially available substance which is used as a topical keratolytic agent, for example, to remove common and plantar warts and to remove scale in psoriasis and other hyperkeratotic and/or scaling dermatoses. Salicylic acid has been incorporated into coal tar-containing shampoos to be applied to the scalp to remove scale and crusts in psoriasis and related conditions.

Psoriasis is a hyperkeratotic dermatologic disease which occurs when new skin cells form at the lower level of the epidermis and move to the upper levels of the epidermis at a rate faster than old skin cells can be shed at the surface. As a result of this and other mechanisms, there are formed papules and plaques of thickened, inflamed skin covered by a layer of flaking dead skin, i.e, scaling. Thus, the signs of psoriasis include abnormal redness due to inflammation, (i.e., erythema), as well as induration and scaling which may appear silvery and laminated. Psoriasis usually involves the scalp and extensor surfaces of the limbs such as the elbows, knees and shins, and the soles of the feet, and the palms of the hands.

Treatments for mild or moderate psoriasis include agents and measures such as topical emollient cream, topical drugs, including corticosteroid preparations, coal tar or anthralin and careful sunbathing. If these agents do not produce an adequate benefit, ultraviolet ("UV") light or the combined use of UV light and an anti-psoriasis drug may be used. Antimetabolites or immunosuppressive agents may be used for difficult or chronic cases that do not respond to usual treatment. The underlying cause of psoriasis is unknown.

In addition to psoriasis, other hyperkeratotic and/or scaly dermatoses are characterized by lichenification/induration, scaling, erythema and pruritus. These include, but are not limited to, diseases such as lichen simplex chronicus, lichenified atopic dermatitis, hyperkeratotic hand dermatitis and non-specific hyperkeratotic chronic dermatitis not otherwise characterized by a specific diagnosis. These dermatoses may occur on the trunk, extremities, scalp and other anatomic locations. Many of the treatments for these diseases include the same medications used to treat psoriasis.

There is no complete cure for psoriasis or for the other hyperkeratotic and/or scaly dermatoses. Thus, there is still a need for a more effective treatment for psoriasis and hyperkeratotic/scaly dermatoses.

### SUMMARY OF THE INVENTION

According to one aspect thereof, the present invention provides a pharmaceutical composition in the form of an ointment for the topical treatment of hyperkeratotic and/or scaly dermatoses which comprises about 1 mg/g of mometasone furoate and about 50 mg/g of salicylic acid and a pharmaceutically acceptable carrier.

In a preferred embodiment, the present invention relates to a pharmaceutical composition wherein the ingredients are present in the following amounts:

| Ingredient | mg/g |
|---|---|
| Mometasone Furoate | about 1.0 |
| Salicylic Acid | about 50.0 |
| Hexylene Glycol | about 120.0 |
| Purified Water | about 30.0 |
| White Wax | about 50.0 |
| Propylene Glycol Stearate | about 20.0 |
| White petrolatum | about 729.0 |

According to another aspect, the present invention provides the use of about 1 mg/g of mometasone furoate and about 50 mg/g of salicylic acid for the preparation of a pharmaceutical composition effective for the topical treatment of psoriasis, such as plaque-type psoriasis vulgaris, hyperkeratotic and/or scaly dermatoses.

### DETAILED DESCRIPTION OF THE INVENTION AND OF THE PREFERRED EMBODIMENTS

The term "treating psoriasis and other hyperkeratotic and/or scaly dermatoses" as used herein means effecting a therapeutic response or improvement in the overall diseases as well as the disease signs including inflammation, erythema, scaling, induration or lesions in mammals especially man afflicted with psoriasis and other hyperkeratotic and/or scaly dermatologic disease. Evaluation of a therapeutic response will be made by clinical experts in psoriasis and other hyperkeratotic diseases.

Applicants have discovered that by incorporation of effective amounts of salicylic acid and of mometasone furoate into a pharmaceutically acceptable ointment vehicle, they were able to effect a therapeutic response in patients afflicted with plaque-type psoriasis vulgaris that was surprisingly better compared to use of a mometasone furoate (0.1%) ointment, alone or a salicylic acid (5%) ointment, alone, or another, corticosteroid ointment, e.g., one containing 0.05% fluocinonide, as the sole active ingredient. (See Tables 1 to 7)

The selection of ingredients for the pharmaceutically acceptable ointment vehicle is critical and the amounts of mometasone furoate (0.1% by weight) and salicylic acid (5% by weight) remain dissolved in the composition at temperatures in the range of 4 to 30°C for at least 24 months. The percentage of mometasone furoate and salicylic acid were monitored at selected intervals to ensure that the actives remained stable and dissolved. The specific ratio of hexylene glycol to water in the pharmaceutical compositions of the present invention is critical to the effective topical treatment of psoriasis and other hyperkeratotic and/or scaly dermatosis. Typically, about 110 to 150 mg of hexylene glycol is admixed with 20 to 40 mg of water and other ingredients as well as the actives to form one gram of pharmaceutical composition. The preferred ratio of hexylene glycol to water is about 4:1.

### METHOD OF MANUFACTURING MOMETASONE FUROATE AND SALICYLICICYLIC ACID OINTMENT FORMULATION OF THE PRESENT INVENTION

1. Into a suitable vessel, charge the white petrolatum USP and heat to 90°C.
2. Upon cooling the petrolatum to 70°C, charge the white wax NF and propylene glycol stearate NF, melting with agitation.
3. To a separate vessel, charge the hexylene glycol NF and purified water USP, mixing with agitation to obtain a homogeneous solution.
4. Heat the solution of Step 3 to 50 - 55°C.
5. Dissolve the salicylic acid USP in approximately 90% of the hexylene glycol/water solution. Check pH (the target pH is approximately 2.0).
6. Dissolve the mometasone furoate micronized in the same solution formed in Step 5.
7. Charge the solution of Step 6 to the melted mixture of Step 2 using homogenization until the batch in the form of an emulsion reaches approximately 40°C.
8. With the remaining hexylene glycol/water solution of Step 3, rinse the vessel which contained the active solution, adding the rinse to the emulsion of Step 7 using homogenization.
9. Cool the emulsion of Step 8 to approximately 25°C while agitating appropriately, until a smooth, uniform ointment is obtained.
10. Transfer the ointment of Step 9 to suitable holding/storage container(s) for filling in appropriate tube/closure system.

Mometasone furoate is available from Schering Corporation, Kenilworth, New Jersey. All of the other above-listed ingredients are commercially available.

In accordance with the above-listed procedure the pharmaceutical compositions of the present invention including the preferred 0.1% mometasone furoate - 5% salicylic acid ointment formulation of the present invention which are listed in Table I below were prepared.

**TABLE I**

| **MOMETASONE FUROATE AND SALICYLICICYLIC ACID OINTMENT** | |
|---|---|
| **Ingredient** | |
| | **mg/g** |
| Mometasone Furoate Micronized | 1.0 |
| Salicylic Acid USP | 50.0 |
| Hexylene Glycol. NF | 120.0 |
| Purified Water USP | 30.0 |
| White Wax NF | 50.0 |
| Propylene Glycol Stearate | 20.0 |
| White Petrolatum USP | 729.0 |

While the preferred topical pharmaceutical composition for practice of the present invention is an ointment, other topical pharmaceutical compositions, including creams, lotions, sprays, gels and aerosols may be prepared in a conventional manner with the aid of one or more carriers or excipients suitable for treating a topical inflammatory condition.

The following four clinical studies were designed to compare the effectiveness and safety of different treatments of plaque-type psoriasis vulgaris; three of the treatments (Studies Nos. 1-3) are applied twice daily ("BID") for 21 days; the fourth treatment (Study No. 4) was applied once daily ("QD") for 21 days.

### Study No. 1

In Study I, three treatments were used: -
1. ELOCON (brand of mometasone furoate 0.1%) ointment.
2. A combination of salicylic acid (3%) and mometasone furoate (0.1%) in the above-described ointment vehicle of Table 1; and
3. A combination of salicylic acid (5%) and mometasone furoate (0.1%) in the ointment vehicle of Table 1.

### STUDY DESIGN AND SYNOPSIS

A single center, randomized, double-blind, balanced incomplete block study was conducted with 60 patients with plaque-type psoriasis vulgaris (Study No. 1). Within each patient, two bilaterally symmetrical lesions were selected as target lesions for treatment and evaluation. The patients were then randomized to receive two of the three treatments indicated below. Each patient, therefore, received a pair of treatments, one treatment per side (right or left side). The three treatments or medications used were: -
- ELOCON brand of mometasone furoate (0.1%) ointment
- 5% Salicylic acid/mometasone furoate (0.1%) ointment of Table 1.
- 3% Salicylic acid/mometasone furoate (0.1%) ointment of Table 1.

Therefore, a third of the patients applied 5% salicylic acid/mometasone furoate (0.1%) ointment and ELOCON (0.1%) ointment , another third applied 3% salicylic acid/mometasone furoate (0.1%) ointment and ELOCON (0.1%) ointment, and the final third applied 3% salicylic acid/mometasone furoate (0.1%) ointment and 5% salicylic acid/mometasone furoate (0.1%) ointment.

Treatments were assigned by random code to one of two similar and bilaterally symmetrical psoriatic target lesions on the patient's trunk, arms or legs. Each pair of treatments occurred together on an equal number of patients. The treatments were applied BID to the target lesions and the non-target lesions for three weeks.

Signs of psoriasis--scaling, erythema, and induration--were graded at the selected target lesions upon entry into the study (initial visit-Day 1) and at follow-up visits on Days 4, 8, 15, and 22 (after 3, 7, 14, and 21 days of treatment) using a severity scale which ranges from 0 = none to 3 = severe (see Table 2). Other areas of psoriatic involvement could be treated; these are designated as non-target areas. Global evaluations of overall change in disease status were made at each follow-up visit for all treated areas (target and non-target lesions), by comparing disease status at each of these visits with the status at the initial visit (see Table 3). In addition, at each follow-up visit, the clinical investigator checked for any local or systemic adverse experiences.

The comparative efficacy of the four treatments were determined on the basis of their effects on individual and total disease sign scores (sum of erythema, induration and scaling) and percent improvement of total disease sign scores of the target lesions, and the global evaluation of change in disease status.

Three additional studies of similar design were also performed and compared (5%) salicylic acid/mometasone furoate, (0.1%) vs. (5%) salicylic acid (Study No. 2); and (5%) salicylic acid/mometasone furoate, (0.1%) vs. fluocinonide ointment, 0.05% (Study No. 3); and (5 %) salicylic acid/mometasone furoate, (0.1%) vs. mometasone furoate (0.1%).

### Investigational Procedures

### A. Application of Drug/Care of Treatment Sites

For application of drug, an amount of the above-listed test medications capable of completely covering the target lesions and all other areas to be treated, maintaining the strict right/left designation, was gently massaged into the skin. Patients were told to wash their hands carefully between applications of treatment to prevent admixture of the two drugs. The first applications of above-listed medications were made by the patient in the doctor's office after the initial evaluation of disease signs. Subsequent treatments were continued to be applied on a BID schedule. Patients were instructed to space the treatments approximately 12 hours apart and apply drugs at least two hours prior to any evaluations.

The patients were cautioned that nothing other than the test medications (including emollients and perfume) was to be applied to the psoriatic lesions during the study (medicated shampoos were allowed). If the patient bathed or showered, the patient was told to do so prior to treatment and not for at least eight hours after treatment application.

**TABLE 2**

| **SCALE FOR THE SIGN SCORES OF PSORIASIS** | |
|---|---|
| Score | Definition |
| 0 | None or absent |
| 1 | Slight or mild; minimal |
| 2 | Moderate or average; easily discernible |
| 3 | Severe or extensive; markedly evident. Half values (e.g., 2.5) may be used, if necessary. |

### 3. GLOBAL EVALUATION

Global evaluations of change in overall disease status in all treated areas were made by the investigator at each follow-up visit. Global evaluations were made relative to the patient's initial overall (Day 1) condition. The scale presented in Table 3 was used.

**TABLE 3**

| **GLOBAL EVALUATION SCALE** | |
|---|---|
| Score | Definition |
| 1 | Cleared = 100% clearance of signs monitored except for some residual discoloration. (No residual erythema must be present). |
| 2 | Excellent = 75% to less than 100% clearance of signs monitored. |
| 3 | Good = 50% to less than 75% clearance of signs monitored. |
| 4 | Fair = 25% to less than 50% clearance of signs monitored. |
| 5 | Poor = Less than 25% clearance of signs monitored. |
| 6 | "Exacerbation" = Flare up of lesions at the target sites. |

### Results - Study No. 1

Based on the percent reduction of total disease sign score, the mometasone furoate (0.1%) salicylic acid (5%) ointment of this invention as shown in Table 4 below was statistically significantly better in treating psoriasis vulgaris than mometasone furoate (0.1%) ointment (single active).

**TABLE 4**

| **PERCENT IMPROVEMENT OF TOTAL SCORES** **(LEAST SQUARES MEANS) FOR STUDY NO. 1** | | | | |
|---|---|---|---|---|
| **TREATMENT** **(BID)** | **DAY 4** | **DAY 8** | **DAY 15** | **DAY 22** |
| ELOCON | | | | |
| Mometasone Furoate (0.1%) Ointment | 23 | 34 | 48 | 58 |
| | | | | |
| Mometasone Furoate (0.1%) and Salicyclic Acid (3%) Ointment | 21 | 37 | 52 | 61 |
| | | | | |
| Mometasone Furoate (0.1 %) and salicylic acid (5%) Ointment | 23 | 39 | 55 | 66 |

The data presented in Table 4 shows a consistent numerical trend favoring mometasone furoate (0.1%) and salicyclic acid (5%) ointment over the other two treatments and favored mometasone furoate (0.1%) and salicyclic acid (3%) ointment over ELOCON ointment, alone. Mometasone furoate (0.1%) with salicylic acid (5%) ointment was statistically significantly better with respect to total improvement in percent improvement in total disease sign scores than ELOCON ointment alone after day 8 visit (P ≤ 0.05)

### STUDY NO. 2

In Study No. 2, two treatments or medications were used:
1. mometasone furoate (0.1%) and salicylic acid (5%) in the pharmaceutical acceptable ointment vehicle of Table I.
2. salicylic acid (5%) in a similar ointment vehicle.

The protocol of Study No. 1 was followed except only forty (40) patients with plaque-type psoriasis vulgaris were treated.

Using the same evaluation criteria used in Study No. 1, the preferred mometasone furoate (0.1%)/salicylic acid (5%) ointment of this invention of Table 1 was statistically significantly better in the effective treatment of psoriasis than salicylic acid (5%) ointment alone as shown in Table 5 below.

**TABLE 5**

| **PERCENT IMPROVEMENT OF TOTAL SCORES** **FOR STUDY NO. 2** | | | | |
|---|---|---|---|---|
| **TREATMENT** **(B I D)** | **DAY 4** | **DAY 8** | **DAY 15** | **DAY 22** |
| Salicyclic acid (5%) Ointment | 19.6 | 26.3 | 29.3 | 28.3 |
| | | | | |
| Mometasone Furoate (0.1%) and salicylic acid (5 %) Ointment | 34.5 | 53.1 | 63.4 | 67.2 |

Statistically significant difference between treatments were seen for all variables at all follow-up visits (P<0.01) favoring mometasone furoate (0.1%)/salicyclic acid (5%) ointment over salicyclic acid ointment alone.

### STUDY NO. 3

In Study No. 3, the following two medications were used:
1. The combination of mometasone furoate (0.1%) and salicylic acid (5%) in the preferred ointment vehicle of the present invention (see Table 1) and
2. LIDEX® (brand of fluocinonide 0.05%) ointment available from Syntex Laboratories, Inc. Palo Alto, California 94303.

The protocol of Study No. 1 was followed except that forty (40) patients with plaque-type psoriasis vulgaris were treated.

Using the same evaluation criteria of Study No. 1, the preferred mometasone furoate (0.1%)/salicylic acid (5%) ointment listed in Table 1 was statistically significantly better than the LIDEX® ointment in the treatment of plaque-type psoriasis vulgaris as shown in Table 6 below.

**TABLE 6**

| **PERCENT IMPROVEMENT OF TOTAL SCORES** **FOR STUDY NO. 3** | | | | |
|---|---|---|---|---|
| **TREATMENT** **(BID)** | **DAY 4** | **DAY 8** | **DAY 15** | **DAY 22** |
| Mometasone Furoate (0.1%) and Salicyclic Acid (5%) Ointment | 12.2 | 32.2 | 52.3 | 65.2 |
| | | | | |
| LIDEX Ointment | 11.7 | 29.4 | 43.3 | 55.3 |

Statistically significant differences (P≤0.05) between the two treatments were seen for all the variables on day 15 and on day 22 of treatment.

### STUDY NO. 4

In Study No. 4, two treatment or medications were applied:
1. Mometasone furoate (0.1%) and salicylic acid (5%) in the pharmaceutical acceptable ointment vehicle of Table I.
2. ELOCON brand of mometasone furoate (0.1%) ointment.

The protocol of Study No. 1 was followed except only forty-one (41) patients with moderate to severe plaque-type psoriasis vulgaris were treated once a day ("QD").

Using the evaluation criteria of Study No. 1, the preferred mometasone furoate (0.1%)/salicylic acid (5%) ointment of this invention listed in Table 1 was statistically significantly better in the effective treatment of moderate to severe psoriasis than mometasone furoate (0.1%) alone as shown below in Table 7.

**TABLE 7**

| **PERCENT IMPROVEMENT OF TOTAL SCORES** **FOR STUDY NO. 4** | | | | |
|---|---|---|---|---|
| **TREATMENT** **(QD)** | **DAY 4** | **DAY 8** | **DAY 15** | **DAY 22** |
| Elocon 0.1% Ointment | 31.7 | 48.6 | 56.2 | 61.2 |
| | | | | |
| Mometasone Furoate (0.1%) and salicylic acid (5%) Ointment | 29.9 | 48.9 | 60.1 | 66.9 |

Statistically significant difference between treatments were seen for percent inprovement in total sign scores and scaling at the Day 22 follow-up visits (P<0.05) favoring mometasone furoate (0.1%)/salicyclic acid (5%) ointment over ELOCON 0.1% ointment alone when each treatment was applied once daily.

Based on an analysis of the Day 22 mean scores for scaling, monetasone furoate (0.1%) and salicylic acid (5%) ointment was statistically significantly more effective (P = 0.03) than memetasone furoate (0.1%) ointment in the reduction of scaling in psoriasis patients when each product was applied once daily, suggesting that the addition of the salicylic acid enhances memetasone furoate efficacy. The adverse event profile for the mometasone furoate (0.1%) salicylic acid (5%) ointment of this invention indicates the combination product of the present invention to be as safe for the treatment of psoriasis as mometasone furoate (0.1%) ointment alone.

Based on the results of the four studies discussed hereinabove, it is expected that other forms of psoriasis and hyperkeratotic and/or scaly dermatoses would be effectively and safely treated in accordance with the present invention.

## Claims

1. A pharmaceutical composition in the form of an ointment for the topical treatment of hyperkeratotic and/or scaly dermatoses which comprises about 1 mg/g of mometasone furoate and about 50 mg/g of salicylic acid and a pharmaceutically acceptable carrier.

2. A pharmaceutical composition according to claim 1, wherein the ingredients are present in the following amounts:
| Ingredient | mg/g |
|---|---|
| Mometasone Furoate | about 1.0 |
| Salicylic Acid | about 50.0 |
| Hexylene Glycol | about 120.0 |
| Purified Water | about 30.0 |
| White Wax | about 50.0 |
| Propylene Glycol Stearate | about 20.0 |
| White petrolatum | about 729.0 |

3. The use of about 1 mg/g of mometasone furoate and about 50 mg/g of salicylic acid for the preparation of a pharmaceutical composition effective for the topical treatment hyperkeratotic and/or scaly dermatoses.

4. The use according to Claim 3 wherein the dermatoses treated is psoriasis.

5. The use according to claim 4 wherein the psoriasis treated is plaque-type psoriasis vulgaris.

## Revendications

1. Composition pharmaceutique sous la forme d'un onguent pour le traitement topique des dermatoses hyperkératosiques et/ou squameuses, qui comprend environ 1 mg de furoate de mométasone et environ 50 mg d'acide salicylique par gramme de composition, et un support pharmaceutiquement acceptable.

2. Composition pharmaceutique selon la revendication 1, qui contient les ingrédients suivants en les quantités indiquées :
| Ingrédient | mg/g |
|---|---|
| Furoate de mométasone | environ 1,0 |
| Acide salicylique | environ 50,0 |
| Hexylèneglycol | environ 120,0 |
| Eau purifiée | environ 30,0 |
| Cire blanche | environ 50,0 |
| Stéarate de propylèneglycol | environ 20,0 |
| Vaseline blanche | environ 729,0 |

3. Utilisation d'environ 1 mg de furoate de mométasone et environ 50 mg d'acide salicylique par gramme de composition, pour la préparation d'une composition pharmaceutique efficace pour le traitement topique des dermatoses hyperkératosiques et/ou squameuses.

4. Utilisation selon la revendication 3, pour laquelle la dermatose traitée est le psoriasis.

5. Utilisation selon la revendication 4, pour laquelle le psoriasis traité est le psoriasis vulgaris du type à plaques.

## Patentansprüche

1. Pharmazeutische Zusammensetzung in Form einer Salbe zur topischen Behandlung hyperkeratotischer und/oder schuppiger Dermatosen, die etwa 1 mg/g Mometasonfuroat und etwa 50 mg/g Salicylsäure und einen pharmazeutisch annehmbaren Träger enthält.

2. Pharmazeutische Zusammensetzung nach Anspruch 1, bei der die Bestandteile in den folgenden Mengen vorhanden sind:
| Bestandteil | mg/g |
|---|---|
| Mometasonfuroat | etwa 1,0 |
| Salicylsäure | etwa 50,0 |
| Hexylenglykol | etwa 120,0 |
| gereinigtes Wasser | etwa 30,0 |
| weißes Wachs | etwa 50,0 |
| Propylenglykolstearat | etwa 20,0 |
| weiße Vaseline | etwa 729,0 |

3. Verwendung von etwa 1 mg/g Mometasonfuroat und etwa 50 mg/g Salicylsäure zur Herstellung einer pharmazeutischen Zusammensetzung, die zur topischen Behandlung hyperkeratotischer und/oder schuppiger Dermatosen wirksam ist.

4. Verwendung nach Anspruch 3, bei der die behandelte Dermatose Psoriasis ist.

5. Verwendung nach Anspruch 4, bei der die behandelte Psoriasis Psoriasis vom Plaque-Typ ist.
